(19) **Europäisches Patentamt / European Patent Office / Office européen des brevets**

(11) **EP 4 410 951 A1**

(12) **EUROPEAN PATENT APPLICATION**
published in accordance with Art. 153(4) EPC

(43) Date of publication:
07.08.2024 Bulletin 2024/32

(21) Application number: 22876844.6

(22) Date of filing: 28.09.2022

(51) International Patent Classification (IPC):
*C12M 1/42* $^{(2006.01)}$  *C12M 1/32* $^{(2006.01)}$
*C12M 3/00* $^{(2006.01)}$  *C12N 13/00* $^{(2006.01)}$
*C12N 5/077* $^{(2010.01)}$  *G01N 33/50* $^{(2006.01)}$
*A61L 27/36* $^{(2006.01)}$  *A61L 27/38* $^{(2006.01)}$

(52) Cooperative Patent Classification (CPC):
A61L 27/36; A61L 27/38; C12M 1/32; C12M 1/42;
C12M 3/00; C12N 5/06; C12N 13/00; G01N 33/50

(86) International application number:
PCT/KR2022/014557

(87) International publication number:
WO 2023/055072 (06.04.2023 Gazette 2023/14)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 30.09.2021 KR 20210130406

(71) Applicant: Youth Bio Global Co., Ltd.
Chungcheongbuk-do 28160 (KR)

(72) Inventors:
• YOO, Seung Ho
Seoul 05607 (KR)
• CHANG, Chi Young
Seoul 08223 (KR)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) **DEVICE AND METHOD FOR FORMING CHONDROPROGENITOR CELL AGGREGATE**

(57) A device for forming a chondroprogenitor cell aggregate according to the present embodiment comprises: a plurality of wells in each of which a micromass is disposed; an electrical stimulation unit which provides electrical stimulation to the micromasses; an image acquisition unit which acquires images by imaging, at predetermined time intervals, the micromasses disposed in the plurality of wells; and a stimulation provision determination unit which determines from the images whether to provide electrical stimulation, wherein the stimulation provision determination unit determines whether to provide the electrical stimulation from a degree of aggregation of the micromass provided with the electrical stimulation.

FIG. 1

## Description

### Technical Field

The present invention relates to a device and a method for forming chondroprogenitor cell aggregates.

### Background Art

Degenerative arthritis occurs when cartilage wears away due to frequent use of the knee and inflammation occurs in the knee joint and ligaments, and 8 out of 10 people over the age of 65 in South Korea suffer from degenerative arthritis. Degenerative arthritis, previously known as a geriatric disease, is now becoming a factor in aggravating arthritis in younger generations due to poor lifestyle habits and an increase in traumatic arthritis due to the trend for exercise. To treat cartilage-related diseases, methods using artificial cartilage containing cartilage cells isolated from costal cartilage are under research.

### Disclosure

### Technical Problem

Knee cartilage is thin, approximately 2-3 mm thick, but serves to facilitate smooth joint movement and prevent joint damage by dispersing impact to surrounding tissues. Knee cartilage, as a representative non-regenerative tissue, can decrease regardless of age due to external factors such as excessive use and damage from external impacts, and can also be damaged by pathological factors such as rheumatoid arthritis.

Degenerative arthritis, which occurs by a decrease of cartilage tissues with age, resulting in exposure of the underlying bones due to an abnormal function of impaired cartilage, restricting movement due to inflammation and pain, and accelerating a decrease in cartilage tissues.

As conventional techniques for addressing joint-related issues, there is provided a method of utilizing stem cells. Since a method of differentiating stem cells into cartilage cells via the addition of relatively expensive growth factors is mainly used, it is necessary to address cost issues for clinical application. However, such cost and immune-related issues are yet to be addressed.

Accordingly, the present invention has been made in view of the above-mentioned problems occurring in the related art. That is, it is an objective of the present invention to provide a device and a method capable of manufacturing therapeutic agents for joint-related diseases, such as degenerative arthritis and rheumatoid arthritis by forming cartilage cells and/or chondroprogenitor cell aggregates using mesenchymal stem cells and fibroblasts.

### Technical Solution

To accomplish the above-mentioned objectives, according to the present invention, there is provided a device for forming chondroprogenitor cell aggregates including: a plurality of wells, in each of which a micromass is arranged; an electrical stimulation unit providing electrical stimulation to the micromasses; an image acquisition unit acquiring images by imaging the micromasses arranged in the plurality of wells at predetermined time intervals; and a stimulation provision determination unit determining from the images whether to provide electrical stimulation, wherein the stimulation provision determination unit determines whether to provide the electrical stimulation from a degree of aggregation of the micromasses provided with the electrical stimulation.

According to an aspect of the device of the present invention, the micromasses are mesenchymal stem cells or fibroblasts.

According to an aspect of the device of the present invention, the mesenchymal stem cells are adipose-derived mesenchymal stem cells.

According to an aspect of the device of the present invention, the electrical stimulation has a frequency up to 20Hz exceeding 0, an oscillation amplitude between -20V and 20V, and a duty cycle of up to 80% exceeding 0.

According to an aspect of the device of the present invention, the electrical stimulation is one or more of a voltage signal and a current signal.

According to an aspect of the device of the present invention, the device provides the electrical stimulation to the micromasses continuously or intermittently within a period of six days.

According to an aspect of the device of the present invention, the stimulation provision determination unit comprises a trained artificial neural network circuit, and the stimulation provision determination unit receives the images and estimates when the micromasses are maximally aggregated to interrupt the electrical stimulation.

According to an aspect of the device of the present invention, when the micromasses are maximally aggregated, the chondroprogenitor cells aggregate in a spheroid shape.

According to an aspect of the device of the present invention, the device further includes: wells in which micromasses stained with fluorescent substances are located; a light source providing light so that the stained micromasses generate fluorescence; and a fluorescent image acquisition unit capturing the micromasses stained with fluorescent substances to obtain images, wherein the electrical stimulation unit additionally provides electrical stimulation to the micromasses stained with the fluorescent substances.

According to an aspect of the device of the present invention, the fluorescent image acquisition unit acquires images of calcium oscillation occurring in the micromasses stained with the fluorescent substances within 40 hours beyond six hours from the provision of the electrical stimulation.

In another aspect of the present invention provided is a method for forming chondroprogenitor cell aggregates including: an electrical stimulation provision operation of providing electrical stimulation to micromasses arranged in a plurality of wells; an image acquisition operation of acquiring images by imaging the micromasses at predetermined time intervals; and a stimulation provision determination operation of determining from the images whether to provide electrical stimulation, wherein the stimulation provision determination operation determines whether to provide the electrical stimulation from a degree of aggregation of the micromasses provided with the electrical stimulation.

According to an aspect of the method of the present invention, the micromasses are mesenchymal stem cells or fibroblasts.

According to an aspect of the method of the present invention, the mesenchymal stem cells are adipose-derived mesenchymal stem cells.

According to an aspect of the method of the present invention, the electrical stimulation provision operation is performed by providing pulse-type electrical stimulation, which has a frequency up to 20Hz exceeding 0, an oscillation amplitude between -20V and 20V, and a duty cycle of up to 80% exceeding 0.

According to an aspect of the method of the present invention, the electrical stimulation is one or more of a voltage signal and a current signal.

According to an aspect of the method of the present invention, the electrical stimulation provision operation provides the electrical stimulation to the micromasses continuously or intermittently within a period of six days.

According to an aspect of the method of the present invention, the stimulation provision determination operation is performed by using a trained artificial neural network circuit, and the stimulation provision determination operation is performed by receiving the images and estimating when the micromasses are maximally aggregated to interrupt the electrical stimulation.

According to an aspect of the method of the present invention, when the micromasses are maximally aggregated, the chondroprogenitor cells aggregate in a spheroid shape.

According to an aspect of the method of the present invention, the method further includes: an operation of providing light so that the stained micromasses generate fluorescence to the wells in which the micromasses stained with fluorescent substances are located; and an operation of acquiring fluorescent images by capturing the micromasses stained with fluorescent substances, wherein electrical stimulation unit is provided to the micromasses stained with fluorescent substances.

According to an aspect of the method of the present invention, the fluorescent image acquisition operation acquires images of calcium oscillation occurring in the micromasses stained with the fluorescent substances within 40 hours beyond six hours from the provision of the electrical stimulation.

According to an aspect of the method of the present invention, the image acquisition operation is performed in parallel with the electrical stimulation provision operation.

## Advantageous Effects

According to the present invention, a device and method capable of forming chondroprogenitor cells by providing electrical stimulation to micromasses are provided.

## Description of Drawings

FIG. 1 is a schematic diagram illustrating an outline of a device for forming chondroprogenitor cell aggregates according to an embodiment of the present invention.

FIG. 2 is a flowchart illustrating an outline of a method for forming chondroprogenitor cell aggregates according to an embodiment of the present invention.

FIG. 3 is a schematic diagram illustrating an example of electrical stimulation provided through electrodes by an electrical stimulation unit.

FIG. 4 is a diagram representing a model of images observed over time when electrical stimulation is applied to micromasses in a culture medium in a well.

FIG. 5 is a diagram illustrating a method for determining an inner closed curve in an image.

FIG. 6 is an example graph illustrating output values of an estimation unit of an electrical stimulation system according to an embodiment of the present invention as time passes.

**Mode for Invention**

Hereinafter, an embodiment of the present invention will be described with reference to the accompanying drawings.

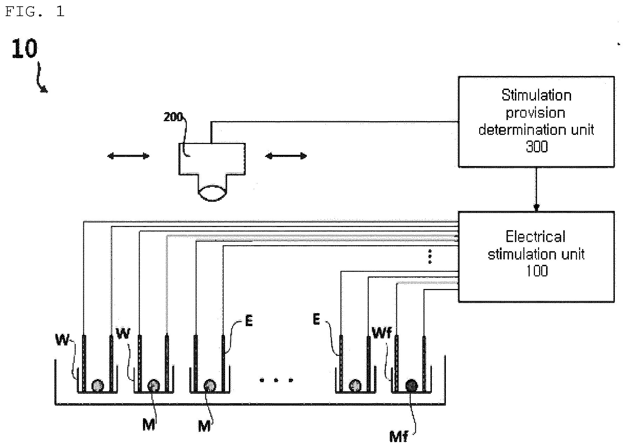

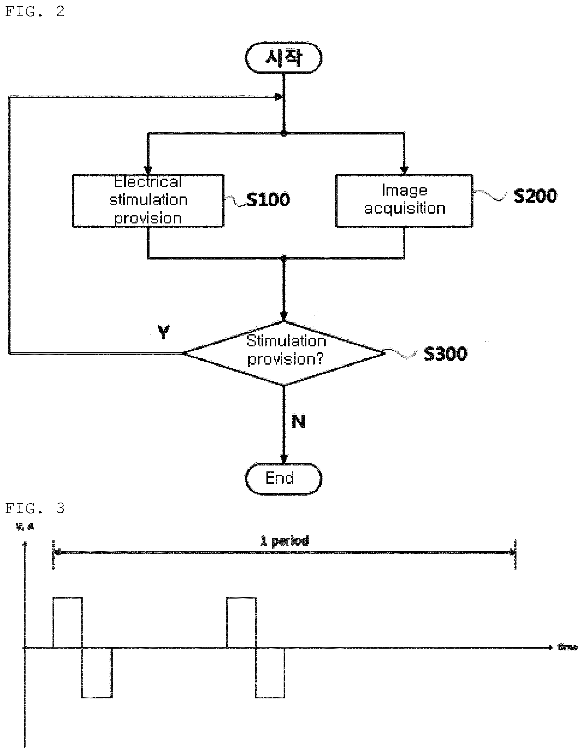

FIG. 1 is a schematic diagram illustrating an outline of a device for forming chondroprogenitor cell aggregates according to an embodiment of the present invention, and FIG. 2 is a flowchart illustrating an outline of a method for forming chondroprogenitor cell aggregates according to an embodiment of the present invention. Referring to FIGS. 1 and 2, the device 10 for forming chondroprogenitor cell aggregates includes: a plurality of wells (W) in each of which a micromass (M) is arranged; an electrical stimulation unit 100 providing electrical stimulation to the micromasses (M); an image acquisition unit 200 acquiring images by imaging the micromasses (M) arranged in the plurality of wells (W) at predetermined time intervals; and a stimulation provision determination unit 300 determining from the images whether to provide electrical stimulation, wherein the stimulation provision determination unit 300 determines whether to provide the electrical stimulation from a degree of aggregation of the micromasses (M) provided with the electrical stimulation.

The method for forming chondroprogenitor cell aggregates according to an embodiment of the present invention includes: an electrical stimulation provision operation (S100) of providing electrical stimulation to micromasses arranged in a plurality of wells; an image acquisition operation (S200) of acquiring images by imaging the micromasses at predetermined time intervals; and a stimulation provision determination operation (S300) of determining from the images whether to provide electrical stimulation, wherein the stimulation provision determination operation (S300) determines whether to provide the electrical stimulation from a degree of aggregation of the micromasses provided with the electrical stimulation.

Micromasses (M) are arranged in the plurality of wells (W). Electrical stimulation is provided to the micromasses during a follow-up process such that the micromasses aggregate to chondroprogenitor cells. In an embodiment, the micromasses (M) may be mesenchymal stem cells or fibroblasts, and the mesenchymal stem cells may be adipose-derived mesenchymal stem cells.

Micromasses (Mf) stained with fluorescent substances may be provided in one or more wells (Wf). The micromasses (Mf) stained with fluorescent substances receive light of a specific wavelength provided by a light source (not illustrated) and emit fluorescence. A fluorescent image acquisition unit (not illustrated) can detect calcium oscillation generated from the micromasses (Mf) stained with fluorescent substances, enable the micromasses (Mf) to function without damage, and infer the status of the remaining micromasses (M). The fluorescent image acquisition unit acquires images of light at wavelengths different from the wavelengths of light detected by the image acquisition unit 200 to detect calcium oscillation.

In an embodiment, the calcium oscillation detection may be performed once or more within 40 hours beyond six hours from the electrical stimulation provision, preferably may be performed periodically within 40 hours beyond six hours from the electrical stimulation provision.

In an embodiment, the micromasses (Mf) may be stained with dyes, and the light source may provide light of wavelengths capable of generating fluorescence by being absorbed by the micromasses (Mf) stained with dyes.

Electrodes (E) are arranged in the wells (W), and the electrodes (E) apply electrical stimulation provided by the electrical stimulation unit 100 to the micromasses (M) located within the wells (W). Additionally, the electrodes (E) may also be positioned in the wells (Wf) where the micromasses (Mf) stained with dyes are located, thereby applying electrical stimulation to the micromasses (Mf) stained with dyes.

The electrical stimulation unit 100 applies electrical stimulation to the micromasses (M) via the electrodes (E). The micromasses (M) receiving electrical stimulation aggregate into a spheroid shape adjacent to the well.

FIG. 3 schematically illustrates an example of electrical stimulation provided through electrodes (E) by the electrical stimulation unit 100. In the example illustrated in FIG. 3, the electrical stimulation may be in the form of pulse-type current signals and/or voltage signals. In the example, the electrical stimulation may be pulse-type signals oscillating with different polarities, but the above is just exemplary, and the pulses may oscillate in a single direction with positive or negative polarity.

In an embodiment, the electrical stimulation may have a frequency up to 20Hz exceeding 0, oscillate between -20V and 20V, and have a duty cycle of up to 80% exceeding 0. The electrical stimulation may be provided continuously or intermittently within a period of six days. However, the above matters are all exemplary embodiments, and the scope of the present invention is not limited thereto.

Referring to FIGS. 1 and 2, as the electrical stimulation unit 100 provides electrical stimulation, the micromasses (M) aggregate into a spheroid shape. The micromasses with undamaged functionality exhibit the calcium ion oscillation that calcium ions flow in and out of the cell membrane. Therefore, it means that observing the calcium ion oscillation confirms that the micromasses are aggregating into chondroprogenitor cells without functional impairment of the micromasses.

The image acquisition unit 200 captures the micromasses (M) arranged in the wells (W) to acquire images. In an embodiment, the image acquisition unit 200 may include an optical system (not illustrated), and an imaging device (not illustrated) converting images formed in the optical system into electrical signals, and capture the micromasses (M) located within the wells (W). For example, the optical system may include lenses such as a convex lenses, and a concave lens, and the imaging device may include one or more of a charge coupled device (CCD) and a CMOS image sensor (CIS). Images captured by the image acquisition unit 200 are provided to the stimulation provision determination unit 300.

The image acquisition unit 200 captures the micromasses (M) arranged in the wells (W) at predetermined intervals to form images, and provides the formed images to the stimulation provision determination unit 300. Images acquired by the image acquisition unit 200 may represent the degree of aggregation of the micromasses (M) arranged in the wells (W) over time.

The stimulation provision determination unit 300 includes a neural network circuit capable of learning and performing inference based on the results of learning. The stimulation provision determination unit 300 having the trained neural network circuit determines whether the electrical stimulation unit 100 will provide electrical stimulation based on the images provided by the image acquisition unit 200.

Hereinafter, a training method of the neural network circuit including a method of determining labels to be used in training will be described.

For the training of the neural network circuit included in the stimulation provision determination unit 300, an expert (human) may observe a plurality of images output by the image acquisition unit 200 in chronological order, and assign a reference label with a specific value to a first image (=reference image), which is the initial image determined as that the generation amount of the chondroprogenitor cells reaches the maximum value, among the plurality of images. For instance, the specific value of the reference label may be 1. That is, the reference label may be associated with images acquired by the image acquisition unit 200 when the amount of chondroprogenitor cells generated by electrical stimulation reaches the maximum amount.

The plurality of images captured may exhibit characteristics according to the following phenomenon. That is, the micromasses (M) contained in the well before applying electrical stimulation may be uniformly distributed in the culture medium contained in the well. As electrical stimulation is provided, the micromasses (M) aggregate and transform into chondroprogenitor cells, and the generated chondroprogenitor cells form clusters.

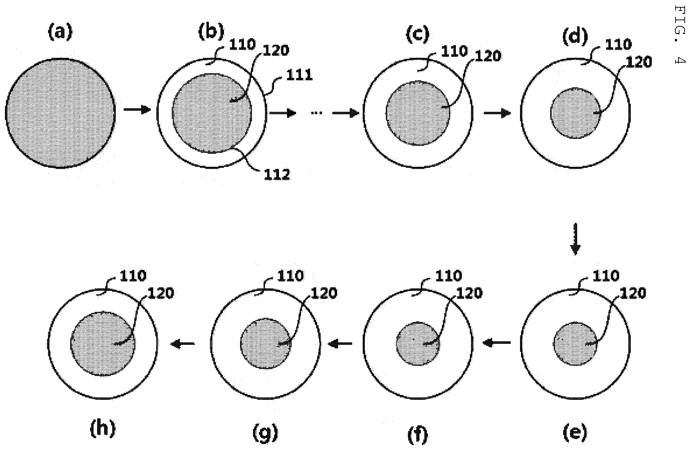

However, if the amount of chondroprogenitor cells increases and electrical stimulation continues to be applied, the aggregated chondroprogenitor cells may disperse again, and ultimately result in a decrease in the amount of chondroprogenitor cells. Such a phenomenon will be further described with reference to FIG. 4.

FIG. 4 is a diagram representing a model of images observed over time when electrical stimulation is applied to the micromasses (M) in the culture medium in the well (W). The large circles depicted in FIGS. 4(a) to 4(h) represent the shape of the outer edge of the well (W) when viewed from above in a case in which the border of the well (W) is circular. FIGS. 4(a) to 4(h) illustrate the results of increased electrical stimulation in a time sequence. FIG. 4(a) indicates the starting point of electrical stimulation. The small circles depicted in FIGS. 4(b) to 4(h) represent the shape of the outer edge of the cluster of chondroprogenitor cells generated by electrical stimulation. Although the actual shape of the cluster of chondroprogenitor cells may not be perfectly circular, it can be roughly modeled as circular. Within the small circles depicted in FIGS. 4(b) to 4(h), not only the generated chondroprogenitor cells but also micromasses (M) may be mixed together. However, since the chondroprogenitor cells are densely packed, it can be considered as the cluster area of the chondroprogenitor cells.

Referring to FIG. 4, in FIG. 4(a), the cluster of chondroprogenitor cells is not observed at the moment when electrical stimulation is provided. From FIG. 4(a) to FIG. 4(e), it can be observed that the area of the cluster of chondroprogenitor cells decreases due to electrical stimulation. Even as the area of the cluster of chondroprogenitor cells decreases, the actual amount of chondroprogenitor cells existing within the cluster gradually increases over time. When the amount of chondroprogenitor cells reaches the maximum value, as illustrated in FIG. 4(e), even if electrical stimulation is applied further, as illustrated in FIG. 4(f), no additional chondroprogenitor cells are generated, and the maximum state can be maintained.

However, if electrical stimulation continues without interruption, as illustrated in FIGS. 4(g) and 4(h), the area of the cluster of chondroprogenitor cells begins to increase again, while in reality, the amount of generated chondroprogenitor cells decreases. Therefore, to maximize the generation of chondroprogenitor cells from the micromasses (M), it is necessary to interrupt the electrical stimulation provided to the the micromasses (M) at an appropriate time. For example, in the operation illustrated in FIG. 4, it is preferable to interrupt the current at the states depicted in FIG. 4(e) and FIG. 4(f). For instance, in operation illustrated in FIG. 4(e), if the current is interrupted, the maximum amount of chondroprogenitor cells can be obtained, the time required to obtain chondroprogenitor cells from micromasses (M) is minimized and power consumption is reduced.

Now, referring to FIGS. 1 and 2, a method for setting the value of the reference label will be described. The value of the reference label assigned to the reference image captured when the amount (number) of chondroprogenitor cells included in the cluster of chondroprogenitor cells has actually reached the maximum can be set to a specific value, 1.

Here, the entity determining that the amount of chondroprogenitor cells has reached the maximum value is an expert (human). The generation amount of the chondroprogenitor cells corresponding to the maximum value can be referred to as the first generation amount, the reference generation amount, or the maximum generation amount, and the reference generation amount can be normalized to the specific value, as mentioned above. For example, the reference generation amount can be presented as 1.

According to an embodiment of the present invention, the stimulation provision determination unit 300 can assign unique label values to each of the plurality of images provided by the image acquisition unit 200, excluding the reference image, according to an algorithm. The label value assigned to each of the remaining images may be less than the specific value of the reference label. For example, the label value assigned to an arbitrary second image among the remaining images may be a value of 1 or less.

The stimulation provision determination unit 300 may determine the label value assigned to each of the remaining images based on the value of the reference label, the reference image, and each captured image, respectively. For example, when the generation amount of the chondroprogenitor cells estimated from the reference image is referred to as the reference generation amount, the generation amount of the chondroprogenitor cells estimated from the second image is referred to as the second generation amount, and the label to be assigned to the second image is referred to as the second label, the relationship of Formula 1 may be established.

【Formula 1】

$$\frac{\text{Second generation amount}}{\text{Second label value}} = \frac{\text{Reference generation amount}}{\text{Reference label value}}$$

In the above Formula 1, the reference label value is a value previously assigned by an expert. For example, the value of the reference label can be 1. Furthermore, the reference generation amount can be a normalized value based on a pre-set value. In an embodiment, regardless of the specific value of the total number of generated chondroprogenitor cells, the generation amount of the chondroprogenitor cells at the maximum generation point determined by the expert can be defined as 1. Additionally, the second generation amount can be a value determined by the method described below. Therefore, the value of the second label that should be assigned to the second image can be determined as shown in the following Formula 2.

【Formula 2】

$$\text{Second label value} = (\text{reference label value}) \times \frac{\text{Second generation amount}}{\text{Reference generation amount}}$$

Generalizing the Formula 2, the value of the $k^{th}$ label for the $k^{th}$ image can be determined as shown in the following Formula 3.

【Formula 3】

$$k^{th} \text{ label value} = (\text{reference label value}) \times \frac{k^{th} \text{ generation amount}}{\text{Reference generation amount}}$$

The method for estimating the generation amount of the chondroprogenitor cells from each image can be presented as follows. First, the stimulation provision determination unit 300 can identify the edge of the well (W) from the image. The well (W) can be a circular or square dish. In this case, the edge of the culture well (W) can be a closed circular curve, as illustrated in FIG. 4.

Continuously, the stimulation provision determination unit 300 can classify the captured images of the micromasses (M) within the well (W) into two areas according to predetermined criteria. The two areas are illustrated in FIG.

4. A first area 110 (outer area) among the two areas may be a doughnut-shaped area with an outer closed curve 111 and an inner closed curve 112. The outer closed curve 111 may be the outer edge of the well (W). Moreover, the second area (inner area, 120) among the two areas may be an area inside the inner closed curve 112. In an embodiment, the inner closed curve 112 can be modeled and defined as being circular or elliptical.

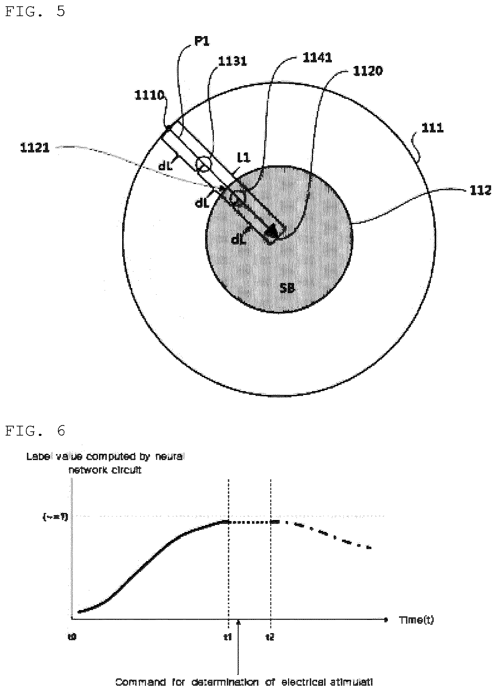

Hereinafter, referring to FIG. 5, a method for determining the inner closed curve in an arbitrary image according to an embodiment of the present invention will be described. The method for determining the inner closed curve 112 in an image may include the following operations. First, the stimulation provision determination unit 300 can determine a path (P1) connecting the outermost point 1110 of the well (W) to the center point 1120 of the well (W) in a captured image. The path (P1) can be a straight line or a curve. When the length of the path (P1) is denoted as L1, a second point can be defined by moving a distance of dL from the outermost point toward the center point.

As described above, consecutive points (k = 1, 2, 3, ...) can be continuously defined by moving a distance of dL from the $k^{th}$ point toward the center point (k is a natural number greater than or equal to 1, dL < L1). The difference value between image attribute values at the $k^{th}$ point and the $(k+1)^{th}$ point can be defined as Dk. The image attribute values may include attributes such as brightness or saturation at each point in an arbitrary image. The selection of specific image attributes does not limit the present invention as long as the attribute values of the image meet the criteria of distinguishing the attributes of images.

As described above, the largest difference value among the difference values Dk (k = 1, 2, 3, ...) defined can be selected. For example, if the difference value D3 is the largest value, it can be considered that the image attribute undergoes the greatest change between a third point 1131 and a fourth point 1141 on the path (P1). Accordingly, it can be determined that a first inner point 1121 of the inner closed curve 112 lies between the third point 1131 and the fourth point 1141.

The process of determining the first inner point 1121 began with selecting the outermost point 1110 of the well (W). When the process of determining the first inner point from various other outer points on the edge of the well (W) is performed, a plurality of different inner points of the inner closed curve 112 can be determined. When the determined multiple inner points are interpolated and connected, the inner closed curve 112 can be completed. As a result, the first area 110 (outer area) and the second area 120 (inner area) can be defined.

If the largest difference value among the difference values Dk (k = 1, 2, 3, ...) is smaller than a pre-determined value, the first inner point 1121 can also be determined as the first outer point 1110. In some cases, the second area 120 (inner area) and the first area 110 (outer area) may not exist. Such a situation may occur, for example, when no electrical stimulation is applied to the micromasses (M). For instance, in FIG. 3 (a), the inner area 120 and the outer area 110 cannot be defined.

The method for determining the first inner point described above is presented as one of various algorithms for distinguishing the second area 120 (inner area), which is approximated as circular or elliptical, from the first area 110 (outer area) existing outside the second area 120 (inner area). That is, the above description is provided to explain the feasibility of dividing the image of the micromass (M) culture solution existing within the well (W) into the two areas, and the present invention is not limited thereto.

The process of dividing the captured image into two areas is performed for each of the plurality of images captured over time and for each micromass (M) contained in the plurality of wells (W). Once the second area 120 (inner area) is defined, an area (SB) of the second area 120 (inner area) can be calculated.

The generation amount of the chondroprogenitor cells estimated from the $k^{th}$ image is referred to as the $k^{th}$ generation amount, and when the area of the second area 120 (inner area) derived from the $k^{th}$ image is referred to as the $k^{th}$ area, the $k^{th}$ generation amount of the chondroprogenitor cells estimated from the $k^{th}$ image may also be defined to be inversely proportional to the $k^{th}$ area. However, depending on the embodiment, although the $k^{th}$ generation amount may be estimated based on the $k^{th}$ area, a rule can also be established in which a nonlinear function intervenes in the relationship between the $k^{th}$ generation amount and the $k^{th}$ area. According to one aspect of the present invention, although the $k^{th}$ generation amount is estimated based on the $k^{th}$ area, the present invention is not limited by the specific functional relationship. As described above, once the $k^{th}$ generation amount is estimated from the $k^{th}$ image, the value of the $k^{th}$ label can be determined using the Formula 3.

Through the above process, electrical stimulation is applied, and then, labels assigned to a series of images capturing the micromasses (M) within the well (W) undergoing changes can be defined or calculated to be obtained.

The stimulation provision determination unit 300 can input the $k^{th}$ image to an input layer of the neural network circuit to update the neural network circuit so that an error between the value output by the neural network circuit and the $k^{th}$ label corresponding to the $k^{th}$ image can be reduced.

In an embodiment, the neural network circuit may be, for example, a convolutional neural network (CNN). The training of the neural network can be repeated using plurality of images and the labels generated for the images. Additionally, the training of the neural network can be repeated using the plurality of images captured and the labels corresponding to the images while applying electrical stimulation to the micromasses (M) arranged in the plurality of wells (W).

In the neural network training method, the input data for training the neural network is the captured data, and it can be considered that a feature of the present invention exists in the method of creating labels corresponding to each training input data.

The timing to block the current provided to the micromass (M) culture solution in the well (W) can be determined. First, the electrical stimulation unit 100 applies current to the well (W) containing the micromass (M) culture solution using a pair of electrodes (E) (S100). Subsequently, the image acquisition unit 200 captures the micromass (M) culture solution at regular time intervals (S200) to obtain images (S200), and then transfers the images to the stimulation provision determination unit 300. The stimulation provision determination unit 300 inputs each transferred captured image into the input layer of the neural network circuit of the stimulation provision determination unit 300.

The stimulation provision determination unit 300 determines whether the electrical stimulation unit 100 will provide electrical stimulation (S300), and outputs a control signal to control the electrical stimulation unit 100. Depending on the control signal output by the stimulation provision determination unit 300, the electrical stimulation unit 100 either provides electrical stimulation or terminates the provision of electrical stimulation. For example, the stimulation provision determination unit 300 may provide a control signal to terminate the current supply when it is determined that the label value determined from the provided image will no longer increase. For this purpose, for instance, the stimulation provision determination unit 300 can observe an average movement value of the label values output by the neural network circuit, and provide a signal to terminate the provision of electrical stimulation at the point at which the average movement value starts to decrease or at the point at which the average movement value saturates for a certain period and indicates no further increase.

In another embodiment, the stimulation provision determination unit 300 can output a label value, which is a scalar label value. The electrical stimulation unit 100 can decide whether to terminate the provision of electrical stimulation based on the history of a series of output values output by the stimulation provision determination unit 300 over time.

For example, the electrical stimulation unit 100 can decide to terminate the current supply when it is determined that the output values output by the stimulation provision determination unit 300 will no longer increase. For this purpose, for instance, a drive current control unit 30 can observe the average movement value of the output values output by an estimation unit 50, and decide to terminate the current supply at the point at which the average movement value starts to decrease or at the point at which the average movement value saturates for a certain period and indicates no further increase.

As described above, referring to FIG. 6, a method of blocking the electrical stimulation provided to the micromass (M) culture solution by the drive current control unit 30 will be described.

FIG. 6 is a graph illustrating output values of an estimation unit of an electrical stimulation system according to an embodiment of the present invention as time passes. The graph in FIG. 6 may represent a prototype value output by the estimation unit 50 as it is, or may represent the prototype value averaged over time intervals.

In FIG. 6, during the time interval from t0 to t1, the label values computed by the neural network circuit increase sequentially, during the time interval from t1 to t2, the label values computed by the neural network circuit are maintained, and during the time interval from t2 onward, the values decrease. As occasion demands, the time interval from t1 to t2 may not practically exist, and the label values may start decreasing directly after t1.

When the curve presented in FIG. 6 is observed over time in real-time, ideally, a command for the termination of the electrical stimulation should be made at t1. However, to determine whether the generation amount of the chondroprogenitor cells has reached the maximum value through observation of the label values computed by the neural network circuit, a little time delay is required. So, the termination of electrical stimulation may practically be commanded after t1.

The exemplary embodiments illustrated in the drawings have been described for facilitating understanding of the invention, but the embodiments of the present invention as described above are only examples. Therefore, it will be appreciated by those skilled in the art that various modifications and equivalent other embodiments are possible from the present invention. Therefore, the actual technical protection scope of the present invention must be determined by the spirit of the appended claims.

**Claims**

1. A device for forming chondroprogenitor cell aggregates, comprising:

    a plurality of wells, in each of which a micromass is arranged;
    an electrical stimulation unit providing electrical stimulation to the micromasses;
    an image acquisition unit acquiring images by imaging the micromasses arranged in the plurality of wells at predetermined time intervals;
    a stimulation provision determination unit determining from the images whether to provide electrical stimulation,

wherein the stimulation provision determination unit determines whether to provide the electrical stimulation from a degree of aggregation of the micromasses provided with the electrical stimulation.

2. The device according to claim 1, wherein the micromasses are mesenchymal stem cells or fibroblasts.

3. The device according to claim 2, wherein the mesenchymal stem cells are adipose-derived mesenchymal stem cells.

4. The device according to claim 1, wherein the electrical stimulation has a frequency up to 20Hz exceeding 0, an oscillation amplitude between -20V and 20V, and a duty cycle of up to 80% exceeding 0.

5. The device according to claim 4, wherein the electrical stimulation is one or more of a voltage signal and a current signal.

6. The device according to claim 1, wherein the device provides the electrical stimulation to the micromasses continuously or intermittently within a period of six days.

7. The device according to claim 1, wherein the stimulation provision determination unit comprises a trained artificial neural network circuit, and
wherein the stimulation provision determination unit receives the images and estimates when the micromasses are maximally aggregated to interrupt the electrical stimulation.

8. The device according to claim 7, wherein when the micromasses are maximally aggregated, the chondroprogenitor cells aggregate in a spheroid shape.

9. The device according to claim 1, further comprising:

wells in which micromasses stained with fluorescent substances are located;
a light source providing light so that the stained micromasses generate fluorescence; and
a fluorescent image acquisition unit capturing the micromasses stained with fluorescent substances to obtain images,
wherein the electrical stimulation unit additionally provides electrical stimulation to the micromasses stained with the fluorescent substances.

10. The device according to claim 9, wherein the fluorescent image acquisition unit acquires images of calcium oscillation occurring in the micromasses stained with the fluorescent substances within 40 hours beyond six hours from the provision of the electrical stimulation.

11. A method for forming chondroprogenitor cell aggregates, comprising:

an electrical stimulation provision operation of providing electrical stimulation to micromasses arranged in a plurality of wells;
an image acquisition operation of acquiring images by imaging the micromasses at predetermined time intervals; and
a stimulation provision determination operation of determining from the images whether to provide electrical stimulation,
wherein the stimulation provision determination operation determines whether to provide the electrical stimulation from a degree of aggregation of the micromasses provided with the electrical stimulation.

12. The method according to claim 11, wherein the micromasses are mesenchymal stem cells or fibroblasts.

13. The method according to claim 12, wherein the mesenchymal stem cells are adipose-derived mesenchymal stem cells.

14. The method according to claim 11, wherein the electrical stimulation provision operation is performed by providing pulse-type electrical stimulation, which has a frequency up to 20Hz exceeding 0, an oscillation amplitude between -20V and 20V, and a duty cycle of up to 80% exceeding 0.

15. The method according to claim 14, wherein the electrical stimulation is one or more of a voltage signal and a current signal.

16. The method according to claim 11, wherein the electrical stimulation provision operation provides the electrical stimulation to the micromasses continuously or intermittently within a period of six days.

17. The method according to claim 11, wherein the stimulation provision determination operation is performed by using a trained artificial neural network circuit, and
wherein the stimulation provision determination operation is performed by receiving the images and estimating when the micromasses are maximally aggregated to interrupt the electrical stimulation.

18. The method according to claim 17, wherein when the micromasses are maximally aggregated, the chondroprogenitor cells aggregate in a spheroid shape.

19. The method according to claim 11, further comprising:

an operation of providing light so that the stained micromasses generate fluorescence to the wells in which the micromasses stained with fluorescent substances are located; and
an operation of acquiring fluorescent images by capturing the micromasses stained with fluorescent substances, wherein electrical stimulation unit is provided to the micromasses stained with fluorescent substances.

20. The method according to claim 19, wherein the fluorescent image acquisition operation acquires images of calcium oscillation occurring in the micromasses stained with the fluorescent substances within 40 hours beyond six hours from the provision of the electrical stimulation.

21. The method according to claim 11, wherein the image acquisition operation is performed in parallel with the electrical stimulation provision operation.

FIG. 1

FIG. 2

FIG. 3

FIG. 4

FIG. 5

FIG. 6

<table>
<tr><td colspan="2" align="center">**INTERNATIONAL SEARCH REPORT**</td><td>International application No.<br>**PCT/KR2022/014557**</td></tr>
</table>

**A. CLASSIFICATION OF SUBJECT MATTER**

**C12M 1/42**(2006.01)i; **C12M 1/32**(2006.01)i; **C12M 3/00**(2006.01)i; **C12N 13/00**(2006.01)i; **C12N 5/077**(2010.01)i; **G01N 33/50**(2006.01)i; **A61L 27/36**(2006.01)i; **A61L 27/38**(2006.01)i

According to International Patent Classification (IPC) or to both national classification and IPC

**B. FIELDS SEARCHED**

Minimum documentation searched (classification system followed by classification symbols)

C12M 1/42(2006.01); A61K 35/32(2006.01); C12M 1/34(2006.01); C12N 5/077(2010.01); G01N 15/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 연골 전구 세포(cartilage progenitor cell), 형성(formation), 마이크로매스 (micromass), 중간엽 줄기세포(mesenchymal stem cell), 섬유아세포(fibroblast), 웰(well), 전기 자극(electric stimulation), 이미지(image), 응집(aggregation), 인공 신경망 회로(artificial neural network), 스페로이드(spheroid)

**C. DOCUMENTS CONSIDERED TO BE RELEVANT**

| Category* | Citation of document, with indication, where appropriate, of the relevant passages | Relevant to claim No. |
|---|---|---|
| Y | KR 10-1603475 B1 (EULJI UNIVERSITY INDUSTRY ACADEMY COOPERATION FOUNDATION) 15 March 2016 (2016-03-15)<br>See claims 1 and 7, and paragraphs [0005], [0017]-[0019], [0028]-[0029], [0036], [0039]-[0041], [0048] and [0053]-[0054]. | 1-21 |
| Y | KR 10-2019-0070096 A (INDUSTRY FOUNDATION OF CHONNAM NATIONAL UNIVERSITY) 20 June 2019 (2019-06-20)<br>See paragraphs [0001], [0020], [0030]-[0031] and [0045], and figures 1-2 and 14. | 1-21 |
| Y | MATTA, C. et al. Calcium signalling in chondrogenesis : implications for catrilage repair. Frontier in Bioscience, Scholar. 2013, vol. 5, pp. 305-324.<br>See page 316. | 10,20 |
| A | KR 10-1653197 B1 (EULJI UNIVERSITY INDUSTRY ACADEMY COOPERATION FOUNDATION) 01 September 2016 (2016-09-01)<br>See entire document. | 1-21 |

☑ Further documents are listed in the continuation of Box C.   ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **17 January 2023** | **18 January 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2022/014557** |

**C.    DOCUMENTS CONSIDERED TO BE RELEVANT**

| Category* | Citation of document, with indication, where appropriate, of the relevant passages | Relevant to claim No. |
| --- | --- | --- |
| A | 권혁준. 사람 중간엽줄기세포의 연골분화를 유도하는 전기자극기반 기술을 이용한 연골재생 세포 치료제 개발. 정부과제 최종보고서. 2019, pp. 1-24 (KWON, Hyuck Joon. Development of cell therapy for cartilage regeneration using electrical stimulation-based technology for chondrogenesis of human mesenchymal stem cells. Government Project Final Report.)<br>        See entire document. | 1-21 |
| A | KR 10-2015-0108963 A (SAMSUNG ELECTRO-MECHANICS CO., LTD. et al.) 01 October 2015 (2015-10-01)<br>        See entire document. | 1-21 |

Form PCT/ISA/210 (second sheet) (July 2022)

**INTERNATIONAL SEARCH REPORT**
Information on patent family members

International application No.

**PCT/KR2022/014557**

| Patent document cited in search report | | | Publication date (day/month/year) | Patent family member(s) | | | Publication date (day/month/year) |
|---|---|---|---|---|---|---|---|
| KR | 10-1603475 | B1 | 15 March 2016 | None | | | |
| KR | 10-2019-0070096 | A | 20 June 2019 | KR | 10-2040638 | B1 | 27 November 2019 |
| KR | 10-1653197 | B1 | 01 September 2016 | US | 11007229 | B2 | 18 May 2021 |
| | | | | US | 2019-0175659 | A1 | 13 June 2019 |
| | | | | WO | 2017-131353 | A1 | 03 August 2017 |
| KR | 10-2015-0108963 | A | 01 October 2015 | US | 2015-0269411 | A1 | 24 September 2015 |
| | | | | US | 9530043 | B2 | 27 December 2016 |

Form PCT/ISA/210 (patent family annex) (July 2022)